# NEUE EUROPÄISCHE PATENTSCHRIFT

(11) **EP 0 320 590 B2**
(45) Veröffentlichungstag und Bekanntmachung des Hinweises auf die Entscheidung über den Einspruch: **08.12.1993**
(45) Hinweis auf die Patenterteilung: 07.08.1991
(21) Anmeldenummer: 88116792.8
(22) Anmeldetag: 10.10.1988
(51) Int. Cl.: A61F 2/12

(54) **Brustprothese**
Mammary prosthesis
Prothèse mammaire

(30) Priorität: 14.12.1987 DE 3742352
(43) Veröffentlichungstag der Anmeldung: 21.06.1989
(73) Patentinhaber: AMOENA Medizin-Orthopädie-Technik GmbH, D-83064 Raubling (DE)
(72) Erfinder: Mulligan, Elisabeth, D-8201 Söllhuben (DE)
(74) Vertreter: Gossel, Hans K., Dipl.-Ing.

(56) Entgegenhaltungen:
- EP-A- 0 054 197
- DE-A- 3 336 279
- US-A- 2 814 808
- US-A- 4 701 230
- Zeitschrift "ärztliche Fortbildung", 65. Jahrgang, Heft 14, Seiten 731 bis 734

## Beschreibung

Die Erfindung betrifft eine Brustprothese nach dem Oberbegriff des Patentanspruchs.

Eine allerdings nur aus einem in Kunststoffolien eingeschweißten schalenförmigen Körper aus einer additionsvernetzenden Zweikomponenten-Silikonkautschuk-Masse bestehende Brustprothese dieser Art ist aus der DE-AS 2605148 bekannt. Diese bekannte Brustprothese erfreut sich bei Frauen allgemein großer Beliebtheit, da die ausgehärtete Zweikomponenten-Silikonkautschuk-Masse in ihrem Gewicht etwa dem Gewicht des natürlichen Brustgewebes entspricht und da der Körper aus der Zweikomponenten-Silikonkautschuk-Masse aufgrund seiner weichen elastischen Nachgiebigkeit und seiner Beweglichkeit die Verhältnisse der natürlichen Brust in nahezu idealer Weise zu simulieren vermag. Die Brustprothesen dieser Art werden zu ihrer Halterung in Büstenhalter eingelegt. Um der in den Büstenhalter eingelegten Brustprothese ein möglichst natürliches Aussehen zu verleihen, ist es bei einer Brustprothese der eingangs beschriebenen Art aus der Praxis bekannt, daß der die konvexe Außenseite bildende Körper aus einer weich eingestellten und der die Rückseite der Prothese bildende flache schalenförmige Körper aus einer härter eingestellten Zweikomponenten-Silikonkautschuk-Masse besteht.

Diese Ausgestaltung hat die Folge, daß die elastisch weiche und unter ihrem Eigengewicht leicht absinkende konvexe Außenseite der Brustprothese durch deren Rückseite aus steiferem Material gestützt wird.

Die bekannte Brustprothese weist nun aber aufgrund der inneren Schicht oder inneren Schale aus härter eingestelltem Zweikomponenten-Silikonkautschuk den Nachteil auf, daß diese innere Schale auf den empfindlichen Narbenbereich drückt und sich diesem nicht in der gebotenen schonenden Weise anpaßt.

Aufgabe der Erfindung ist es daher, eine Brustprothese der eingangs angegeben Art zu schaffen, deren Tragekomfort dadurch wesentlich erhöht ist, daß sie sich gut dem Narbenbereich anpaßt.

Erfindungsgemäß wird diese Aufgabe bei einer Brustprothese nach dem Oberbegriff des Anspruchs durch dessen Kennzeichnendes Merkmal gelöst. der gattungsgemäßen Art dadurch gelöst, daß der äußere, der äußeren Brustform nachgebildete Körper eine Härte besitzt, die der weich elastischen Nachgiebigkeit des natürlichen Brustgewebes angeglichen ist, und daß der innere Körper eine weiche, gelartige Konsistenz aufweist. Bei der erfindungsgemäßen Brustprothese ist entgegen der bisherigen Meinung, daß der vordere Bereich der Prothese durch einen hinteren schalenartigen Teil größerer Härte gestützt werden müsse, gerade dieser Teil aus weicherem Material von gelartiger Konsistenz hergestellt. Der innere schalenartige Teil weist somit aufgrund seiner gelartigen Konsistenz eine so große Weichheit auf, daß er sich dem Narbenbereich anpaßt und auf diesen keinen Druck mehr ausübt. Damit sind die Trageeigenschaften der Brusprothese wesentlich verbessert worden. Überraschenderweise hat sich gezeigt, daß trotz der weicheren Ausgestaltung der inneren Schale das Aussehen der in den Büstenhalter eingelegten Brustprothese nicht leidet und diese ihr natürliches Aussehen beibehält.

Die erfindungsgemäße Brustprothese läßt sich dadurch herstellen, daß zwischen die elastischen, die Brustprothesen bildenden Kunststoffolien eine Zwischenfolie eingelegt und diese gemeinsam mit den äußeren Folien längs des späteren Brustprothesenrandes bis auf je eine Einfüllöffnung verschweißt wird, daß zunächst zwischen die die konvexe Außenseite der Brustprothese bildende Folie und die Zwischenfolie eine weich eingestellte Zweikomponenten-Silikonkautschuk-Masse und zwischen die beiden anderen Folien ein deren Verschweißen oder Verblocken verhinderndes Trennmittel eingefüllt werden, das nach dem Schließen der Form mit dem oberen Formteil, dessen Kontur der inneren Seite einer Brustprothese aussteifenden Schale entspricht, die Masse ausgehärtet wird, daß anschließend zwischen die anderen Folien eine gelartig eingestellte Zweikomponenten-Silikonkautschuk-Masse eingefüllt und nach dem Schließen mit dem oberen Formteil, dessen Kontur der Prothesenrückseite entspricht, die durch die zweite Füllung gebildete Schale ausgehärtet wird.

Die die schalenförmigen Körper einfassenden Kunststoffolien bestehen zweckmäßigerweise aus Polyurethanfolien.

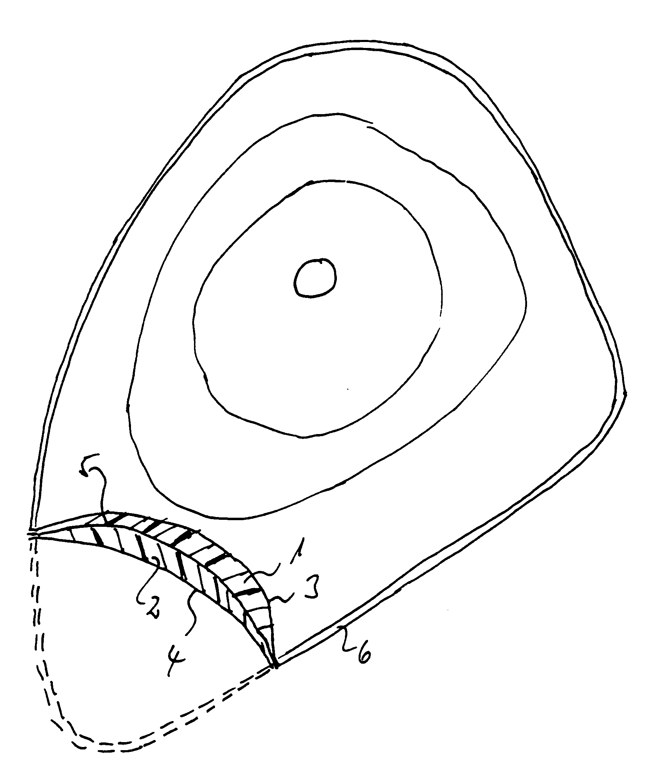

Ein Ausführungsbeispiel der Erfindung wird nachstehend anhand der Zeichung, in deren einziger Figur eine Brustprothese, teilweise im Schnitt, dargestellt ist, näher erläutert.

In der Zeichnung ist eine Brustprothese dargestellt, deren einer Teil weggenschnitten ist. Die Brustprothese besteht aus einem äußeren konvexen Körper 1 aus einer weich eingestellten additionsvernetzenden Zweikomponenten-Silikonkautschuk-Masse und einem die Prothesenrückseite bildenden Körper 2 in Form einer flachen Schale, die aus einer weicher eingestellten, gelartigen Masse aus additionsvernetzendem Zweikomponenten-Silikonkautschuk besteht. Die beiden Körper 1,2 sind auf ihren Außenseiten von Polyurethanfolien 3,4 eingefaßt und voneinander durch eine Zwischenfolie 5, ebenfalls aus Polyurethan, getrennt. Alle drei Folien sind längs des Prothesenrandes durch eine gemeinsame Schweißnaht 6 miteinander verbunden.

## Patentansprüche

1. Brustprothese, bestehend aus zwei schalenförmigen Körpern aus einer gelartigen additionsvernetzenden Zweikomponenten-Silikonkautschuk-Masse unterschiedlicher Weichheit, deren die Außen- und Innenseite abdeckenden Kunststoffolien mit einer die beiden Körper trennenden, mittleren Kunststoffolie längs eines gemeinsamen umlaufenden Randes miteinander verschweißt sind, beider der äußere, der Brustform nachgebildete Körper (1) eine Härte besitzt, die der weich elastischen Nachgiebigkeit des natürlichen Brustgewebes angeglichen ist, dadurch gekennzeichnet daß der innere Körper (2) eine weichere Konsistenz aufweist, so daß er sich aufgrund seines weichheit dem Narbenbereich anpassen kann und auf diesen keinen Druck mehr ausübt.

## Claims

1. Breast prosthesis, consisting of two shell-shaped bodies of a gel-like addition-crosslinked two-component silicone rubber compound of varying softness, of which the plastics films covering the outside and inside are welded to each other along a common peripheral edge by a middle plastics film separating the two bodies, in which the outer body (1), simulating the breast shape, has a hardness which is made to match the softly resilient compliance of the natural breast tissue, characterised in that the inner body (2) has a softer consistency, so that, owing to its softness, it can adapt to the scar area and no longer exerts any pressure on the latter.

## Revendications

1. Prothèse mammaire composée de deux corps en forme de coquilles, formés par une masse de caoutchouc au silicone réticulant d'addition ressemblant à du gel, de mollesses différentes, dont les films en matière plastique recouvrant les faces extérieure et intérieure sont reliés par soudage l'un à l'autre et à un film en matière plastique médiane, séparant les deux corps, le long d'un bord commun périphérique,
dans laquelle
le corps extérieur (1) reproduisant la forme du sein présente une dureté qui est adaptée à la flexibilité élastique molle du tissu de sein naturel,
caractérisée en ce
que le corps intérieur (2) présente une consistance plus molle, de sorte qu'en raison de sa mollesse il peut s'adapter à la zone de cicatrice et n'exerce plus de pression sur celle-ci.
